# EUROPEAN PATENT APPLICATION

(11) **EP 3 677 190 A1**
(43) Date of publication of application: **08.07.2020**
(21) Application number: 19150616.1
(22) Date of filing: 07.01.2019
(51) Int. Cl.: A61B 9/00

(54) **GENERATION OF A DATASET RELATED TO A TENDON REFLEX RESPONSE OF A SUBJECT**

(71) Applicant: Nokia Technologies Oy, 02610 Espoo (FI)
(72) Inventor: MÜLLER, Kiti, 00430 Helsinki (FI); LINDHOLM, Harri, 00320 Helsinki (FI)
(74) Representative: Whiting, Gary

(57) **Abstract**

An apparatus, method and computer program is described comprising: receiving one or more first signals indicative of an impact between a striking means and a tendon of a subject, caused by a strike by the striking means on the subject; receiving one or more second signals indicative of a reflex response to said impact; and generating a dataset based on one or more data elements obtained from the one or more first signals and the one or more second signals.

## Description

### Field

The present specification relates to the generation of datasets based on data related to a reflex response caused by a strike by a striking means (such as a reflex hammer) on a subject.

### Background

The examination of tendon reflexes is part of routine clinical procedure. Reflexes are elicited by tapping with a device, such as a reflex hammer, at certain areas of a person (e.g. near upper and lower limb joints). Reflexes may be accelerated in patients with upper motor neuron problems and may be decreased or missing in patients with peripheral nerve diseases. There remains a need for alternative approaches in this field.

### Summary

In a first aspect, this specification describes an apparatus (such as a control module or a server) comprising: means for receiving one or more first signals indicative of an impact between a striking means (such as a hammer) and a tendon of a subject (such as a patient), caused by a strike by the striking means on the subject; means for receiving one or more second signals indicative of a reflex response to said impact; and means for generating a dataset based on one or more data elements obtained from the one or more first signals and the one or more second signals.

The one or more first signals may be received from one or more first sensors and the one or more second signals may be received from one or more second sensors. The one or more first sensors may comprise: an imaging device; one or more sensors (such as pads, as discussed in detail below) attached to the subject; and/or form part of said striking means. The one or more second sensors may comprise one or more of: an imaging device; and one or more sensors (e.g. pads or a toe cap) attached to the subject. At least some of the first and second sensors may be the same sensor (e.g. the hammer and the imaging device referred to above are examples of both a first sensor and a second sensor). At least some of said sensors may provide one or more of a timer data and a clock signal for recording time data (e.g. for data synchronization purposes) associated with said signals. By way of example, time data may be transmitted with the sensor data. The time data may be recorded at the sensor, but this is not essential to all embodiments.

The one or more data elements may comprise a first information of an impact location between the striking means and the tendon. The first information may be based on data received from one or more of the first sensors. The impact location may be a patella tendon of the subject.

The one or more data elements may comprise a second information of impact force, number of strikes, time of one or more strikes and/or duration between respective strikes. The second information may be based on data received from one or more of the first sensors.

The one or more data elements may comprise a third information of speed of the striking means and/or direction of movement of striking means. The third information may be based on data received from one or more of the first sensors.

The one or more data elements may comprise a fourth information of a magnitude of the reflex response, a reflex location (e.g. an ankle, toe and/ or a foot of the subject) of the reflex response and/ or a time of the reflex response. The fourth information may be based on data received from one or more of the second sensors.

The one or more data elements comprise a fifth information of a duration between the impact and the reflex response. The fifth information may be based on data from one or more of the first sensors and/or second sensors.

Some embodiments comprise means for transmitting said dataset, such as a communication means. The communication means may include a wireless module. Alternatively, or in addition, some embodiments comprise means for storing said dataset (e.g. in a data logger).

The said means may comprise: at least one processor; and at least one memory including computer program code, the at least one memory and the computer program configured, with the at least one processor, to cause the performance of the apparatus.

In a second aspect, this specification describes a method comprising: receiving one or more first signals indicative of an impact between a striking means (such as a hammer) and a tendon of a subject (such as a patient), caused by a strike by the striking means on the subject; receiving one or more second signals indicative of a reflex response to said impact; and generating a dataset based on one or more data elements obtained from the one or more first signals and the one or more second signals.

The one or more first signals may be received from one or more first sensors (such as an imaging device, one or more sensors attached to the subject and/or may form part of the striking means) and the one or more second signals maybe received from one or more second sensors (such as an imaging device and/or one or more sensors attached to the subject).

The one or more data elements may take many forms, such as: a first information of an impact location between the striking means and the tendon; a second information of impact force, number of strikes, time of one or more strikes and/or duration between respective strikes; a third information of speed of the striking means and/or direction of movement of striking means; a fourth information of a magnitude of the reflex response, a reflex location (e.g. an ankle, toe and/or a foot of the subject) of the reflex response and/or a time of the reflex response; and/or a fifth information of a duration between the impact and the reflex response.

Some embodiments comprise transmitting said dataset, for example using a wireless module. Alternatively, or in addition, some embodiments comprise storing said dataset (e.g. in a data logger).

In a third aspect, this specification describes any apparatus configured to perform any method as described with reference to the second aspect.

In a fourth aspect, this specification describes computer-readable instructions which, when executed by computing apparatus, cause the computing apparatus to perform any method as described with reference to the second aspect.

In a fifth aspect, this specification describes a computer program comprising instructions for causing an apparatus to perform at least the following: receive one or more first signals indicative of an impact between a striking means and a tendon of a subject, caused by a strike by the striking means on the subject; receive one or more second signals indicative of a reflex response to said impact; and generate a dataset based on one or more data elements obtained from the one or more first signals and the one or more second signals.

In a sixth aspect, this specification describes a computer-readable medium (such as a non-transitory computer readable medium) comprising program instructions stored thereon for performing at least the following: receiving one or more first signals indicative of an impact between a striking means (such as a hammer) and a tendon of a subject (such as a patient), caused by a strike by the striking means on the subject; receiving one or more second signals indicative of a reflex response to said impact; and generating a dataset based on one or more data elements obtained from the one or more first signals and the one or more second signals.

In a seventh aspect, this specification describes an apparatus comprising: at least one processor; and at least one memory including computer program code which, when executed by the at least one processor, causes the apparatus to: receive one or more first signals indicative of an impact between a striking means and a tendon of a subject, caused by a strike by the striking means on the subject; receive one or more second signals indicative of a reflex response to said impact; and generate a dataset based on one or more data elements obtained from the one or more first signals and the one or more second signals.

In an eighth aspect, this specification describes an apparatus (such as a control module or a server) comprising: a first input for receiving one or more first signals indicative of an impact between a striking means (such as a hammer) and a tendon of a subject (such as a patient), caused by a strike by the striking means on the subject; a second input for receiving one or more second signals indicative of a reflex response to said impact; and a control module (such as a processor) for generating a dataset based on one or more data elements obtained from the one or more first signals and the one or more second signals. Some embodiments comprise a wireless module (or some other communication device) for transmitting said dataset. Alternatively, or in addition, some embodiments comprise a memory (such as a data logger) for storing said dataset.

### Brief description of the drawings

Example embodiments will now be described, by way of non-limiting examples, with reference to the following schematic drawings, in which:
FIG. 1 shows a system in which an example embodiment maybe used;
FIG. 2 shows a sketch showing a number of example places on a subject's body where tendon reflexes can be elicited;
FIG. 3 is a block diagram of a system in accordance with an example embodiment;
FIG. 4 shows a sensing pad in accordance with an example embodiment;
FIG. 5 shows a movement sensor in accordance with an example embodiment;
FIG. 6 shows a system in which an example embodiment may be used;
FIG. 7 is a flow chart showing an algorithm in accordance with an example embodiment;
FIG. 8 is a block diagram of components of a system in accordance with an example embodiment; and
FIGS. 9A and 9B show tangible media, respectively a removable memory unit and a compact disc (CD) storing computer-readable code which when run by a computer perform operations according to example embodiments.

### Detailed description

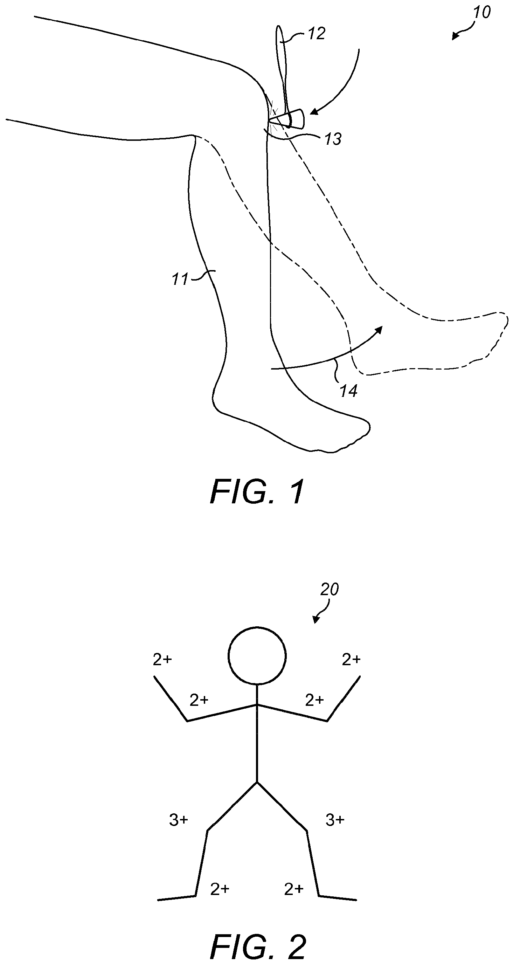

FIG. 1 shows a system, indicated generally by the reference numeral 10, in which an example embodiment may be used. The system 10 comprises a leg 11 that is being struck by a reflex hammer 12. As indicated in the system 10, the reflex hammer 12 is striking the leg 11 at a patella tendon 13 of the leg. In response to the hammer strike, a reflex action of the foot (indicated by the arrow 14) occurs.

FIG. 2 is a sketch, indicated generally by the reference numeral 20, showing a number of example places on a subject's body where tendon reflexes can be elicited, together with subjectively estimated levels of reflex strengths of an example subject. A shorthand scale records reflex responses at various points on a subject as follows:
- 0: response absent
- 1+: response hypoactive
- 2+: response normal
- 3+: response hyperactive
- 4+: response hyperactive with clonus
- 5+: sustained clonus response

Thus, the example responses shown in FIG. 2 relate to a patient in which patella reflex responses were judged to be hyperactive and other reflex responses were judged to be normal.

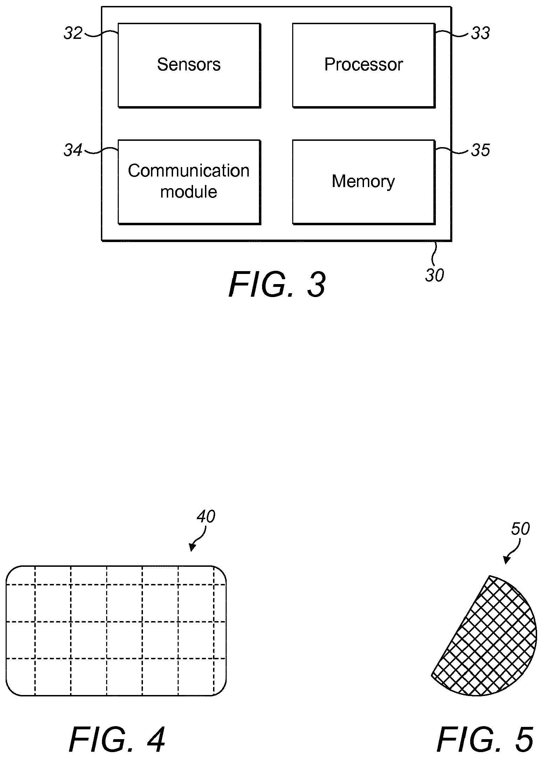

FIG. 3 is a block diagram of a system, indicated generally by the reference numeral 30, in accordance with an example embodiment. The system 30 comprises one or more sensors 32, a processor 33, a communication module 34 and a memory module 35 (such as a data-logger). The communication module 34 may, for example, be a wired or a wireless module.

The system 30 may be implemented as part of a striking means (similar to the reflex hammer 12 described above). Alternatively, some of the features of the system 30 (such as at least some of the sensors 32) may be implemented as part of a striking means, with other features being implemented elsewhere (and being in communication with the striking means).

The sensors 32 are provided to receive or generate one or more first signals indicative of an impact between a striking means (such as a reflex hammer) and a tendon of a subject (e.g. a patient), caused by a strike by the striking means on the subject. Such sensors may, for example, record one or more of the speed, direction and force with which the striking means strikes or taps the tendon.

The sensors 32 may also receive or generate one or more second signals indicative of a reflex response to said impact.

The processor 33 generates a dataset based on one or more data elements obtained from the one or more first signals and the one or more second signals. Thus, the dataset is generated based on signals indicative of an impact between a striking means and a subject and signals indicative of a reflex response to said impact. At least some of the sensors may provide timing information (for example in the form of a timer output or a clock signal).

The communication module 34 is an example of a transmission means that maybe provided for transmitting the dataset generated by the processor 33. The memory module 34 is an example of a means that may be provided for storing said dataset. In some embodiments, one or both of the communication module 34 and the memory module 35 maybe omitted.

FIG. 4 shows a sensing pad, indicated generally by the reference numeral 40, in accordance with an example embodiment. The sensing pad 40 maybe attached to a subject (e.g. on the skin of the subject). The sensing pad 40 may obtain data relating to one or more strikes (for example by a striking hammer). Thus, the sensing pad 40 may obtain data relating to a force of individual strikes and/or strike timings. Alternatively, or in addition, the sensing pad 40 may enable a location of a strike to be determined. Location determination may be achieved using a plurality of force sensors that enable different force locations to be determined. Alternatively, or in addition, location determination may be achieved by providing position markers, which position markers may, for example, be detectable by an imaging device.

FIG. 5 shows a movement sensor, indicated generally by the reference numeral 50, in accordance with an example embodiment. The movement sensor 50 may include means for determining a position of the sensor 50. By placing the sensor 50 on a toe of a subject (for example), a position of the toe can be monitored. The movement sensor 50 may itself determine position (or relative position); alternatively, or in addition, the movement sensor 50 may be used in conjunction with an imaging device to determine position.

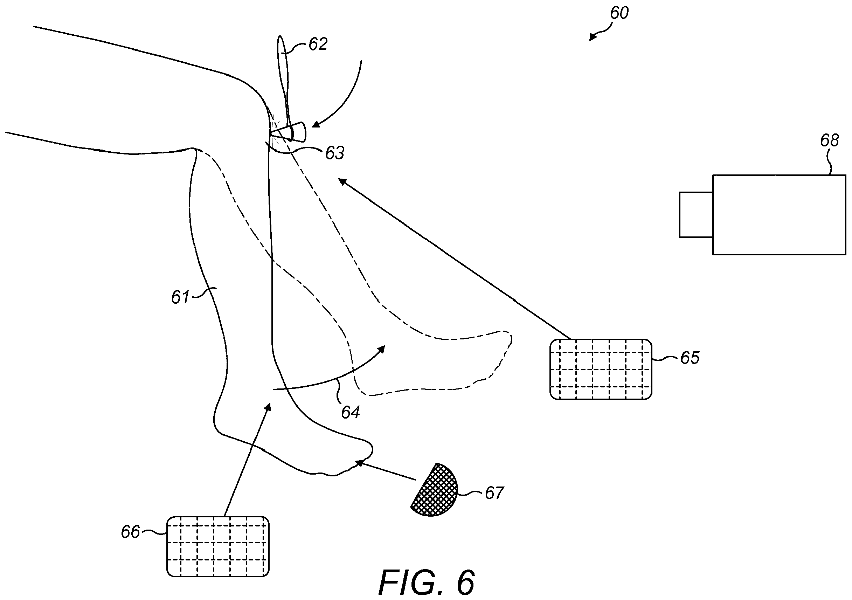

FIG. 6 shows a system, indicated generally by the reference numeral 60, in which an example embodiment may be used. The system 60 comprises a leg 61 that is being struck by a reflex hammer 62. As indicated in the system 60, the reflex hammer 62 is striking the leg 61 at a patella tendon 63 of the leg. In response to the hammer strike, a reflex action of the foot (indicated by the arrow 64) occurs.

The system 60 includes a number of sensors that can be used for monitoring impacts of the striking hammer and a subject (e.g. the leg 61) and for monitoring a reflex response to such impacts. The sensors of the system 60 are provided by way of example only. At least some of those sensors may be omitted from some example embodiments and other sensors (not shown in FIG. 6) may be included in some example embodiments. Moreover, different striking location may be used and sensors may be provided at different locations to monitor different reflex responses in response to different strikes.

The sensors of the system 60 comprise a first sensing pad 65, a second sensing pad 66, a sensing toe cap 67 and an imaging device 68. The first and second sensing pads may be similar to the sensing pad 40 described above. The sensing toe cap 67 may be similar to the movement sensor 50 described above.

Of course, although the systems 10 and 60 show patella tendons being struck in order to elicit a reflex response, the principles described herein can be used elsewhere on a subject's body where tendon reflexes can be elicited (such as the points shown in the sketch 20 described above).

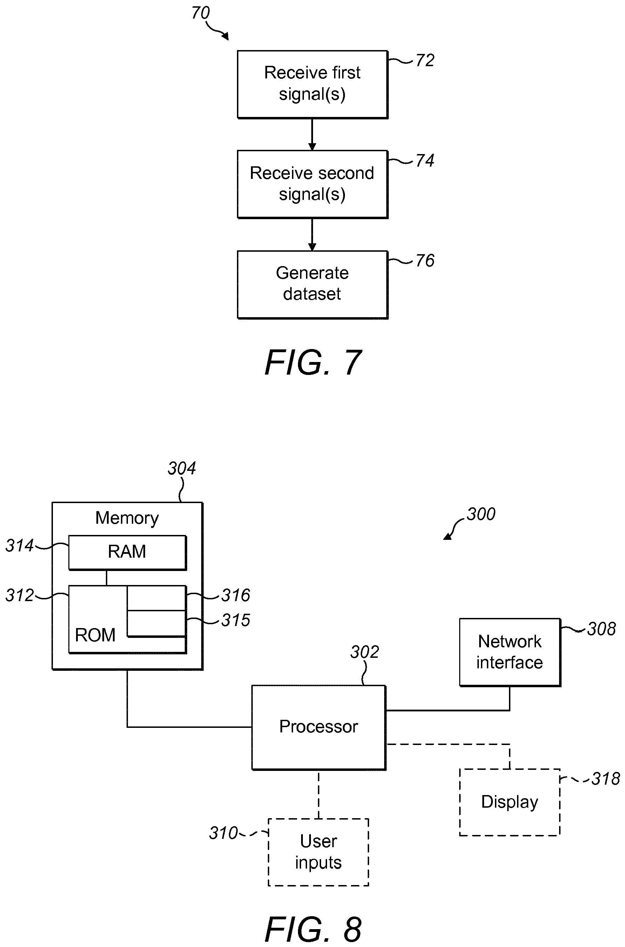

FIG. 7 is a flow chart showing an algorithm, indicated generally by the reference numeral 70, in accordance with an example embodiment. The algorithm 70 may, for example, be implemented at the system 60.

The algorithm 70 starts at operation 72, where one or more first signals indicative of an impact between a hammer (or some other striking means) and a tendon of a subject, caused by a strike by the hammer on the subject are received or obtained. The one or more first signals may be received from one or more first sensors, such as: an imaging device (such as the imaging device 68 described above); one or more sensors attached to the subject (e.g. pads, such as the sensing pads 40, 65 and 66 described above); and/or a part of the hammer itself.

At operation 74, one or more second signals indicative of a reflex response to said impact are received or obtained. The one or more second signals may be received from one or more second sensors, such as one or more of an imaging device and/or one or more sensors attached to the subject. Example sensors include pads, such as the sensing pad 40 described above, movement sensors, such as the movement sensor 50 described above, or the toe cap 67 described above, and an imaging device, such as the imaging device 68 described above.

At least some of the first sensors providing the first signals described above and the second sensors providing the second signals described above may be the same sensors. For example, the hammer (or other striking means) described above may include sensors that are examples of both a first sensor and a second sensor.

At operation 76, a dataset based on one or more data elements obtained from the one or more first signals and the one or more second signals is generated. The data elements included in the dataset in the operation 76 may take many forms, some of which are described below by way of example. Of course, any combination of the examples below could be provided (optionally in combination with other data elements not described below).

By way of example, the one or more data elements may include at least some of the following:
- A first information of an impact location between the striking means and the tendon. The impact location may, for example, be a patella tendon (or some other tendon) of the subject (as shown in the example of FIG. 6). The first information may be based on data received from one or more of the first sensors described above.
- A second information of impact force, number of strikes, a time of one or more strikes and/or a duration between strikes. The second information may be based on data received from one or more of the first sensors described above.
- A third information of speed of the striking means and/or direction of movement of striking means. The third information may be based on data received from one or more of the first sensors described above.
- A fourth information of a magnitude of a reflex response, a reflex location of a reflex response, a time of one or more reflex responses and/or a duration between reflex responses. The reflex location may, for example, be an ankle, toe and/or a foot of the subject. The fourth information may be based on data received from one or more of the second sensors described above.
- A fifth information of a duration between impact(s) and reflex response(s) (for example, indicative of a delay between initiation of a reflex and the subsequent reflex response). The fifth information may be based on data received from one or more of the first and/or second sensors described above.

At least some of the sensors described above may provide one or more of a timer data and a clock signal for recording time data (e.g. for data synchronization purposes) associated with said signals. For example, time data maybe transmitted with the sensor data. The time data may be recorded at the sensor, but this is not essential to all embodiments. The time data may be used in the generation of the fifth information described above.

In the embodiments described above, a tool (such as a hammer) is used as the striking means used to provide an impact between a striking means and a tendon of a subject. This is not essential to all embodiments. For example, a hand of a medical professional (or some other person) carrying out a test could be used to strike the relevant tendon. In such an embodiment, the person striking the tendon may wear a glove or similar means comprising sensors in order to generate one or more first signals indicative of the impact between the hand applying the striking force and the tendon of the subject. Alternatively, or in addition, a sensor patch maybe attached to the hand (or glove) applying the striking force. The sensor patch may, for example, be similar to the sensing pads 40, 65 and 66 described above.

For completeness, FIG. 8 is a schematic diagram of components of one or more of the example embodiments described previously, which hereafter are referred to generically as processing systems 300. A processing system 300 may have a processor 302, a memory 304 closely coupled to the processor and comprised of a RAM 314 and ROM 312, and, optionally, user input 310 and a display 318. The processing system 300 may comprise one or more network/apparatus interfaces 308 for connection to a network/apparatus, e.g. a modem which maybe wired or wireless. Interface 308 may also operate as a connection to other apparatus such as device/apparatus which is not network side apparatus. Thus direct connection between devices/apparatus without network participation is possible.

The processor 302 is connected to each of the other components in order to control operation thereof.

The memory 304 may comprise a non-volatile memory, such as a hard disk drive (HDD) or a solid state drive (SSD). The ROM 312 of the memory 314 stores, amongst other things, an operating system 315 and may store software applications 316. The RAM 314 of the memory 304 is used by the processor 302 for the temporary storage of data. The operating system 315 may contain code which, when executed by the processor implements aspects of the algorithm 70 described above. Note that in the case of small device/ apparatus the memory can be most suitable for small size usage i.e. not always hard disk drive (HDD) or solid state drive (SSD) is used.

The processor 302 may take any suitable form. For instance, it may be a microcontroller, a plurality of microcontrollers, a processor, or a plurality of processors.

The processing system 300 may be a standalone computer, a server, a console, or a network thereof. The processing system 300 and needed structural parts may be all inside device/ apparatus such as IoT device/ apparatus i.e. embedded to very small size

In some example embodiments, the processing system 300 may also be associated with external software applications. These may be applications stored on a remote server device/ apparatus and may run partly or exclusively on the remote server device/ apparatus. These applications maybe termed cloud-hosted applications. The processing system 300 may be in communication with the remote server device/ apparatus in order to utilize the software application stored there.

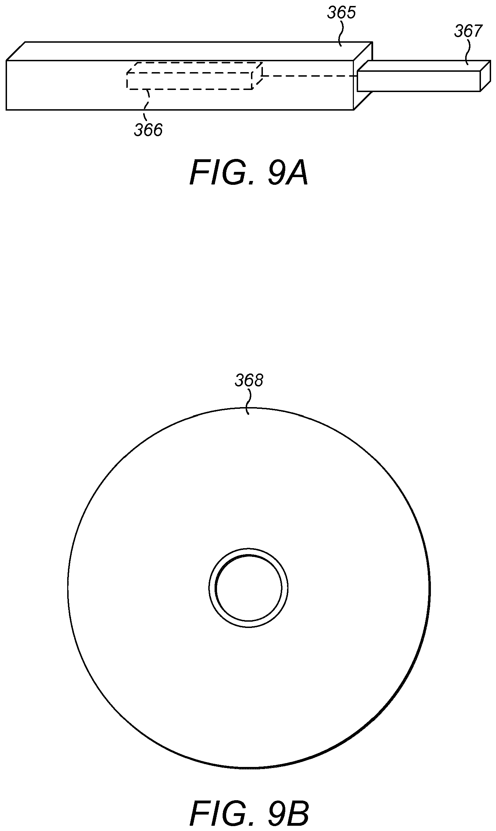

FIGS. 9A and 9B show tangible media, respectively a removable memory unit 365 and a compact disc (CD) 368, storing computer-readable code which when run by a computer may perform methods according to example embodiments described above. The removable memory unit 365 may be a memory stick, e.g. a USB memory stick, having internal memory 366 storing the computer-readable code. The memory 366 may be accessed by a computer system via a connector 367. The CD 368 may be a CD-ROM or a DVD or similar. Other forms of tangible storage media may be used. Tangible media can be any device/apparatus capable of storing data/information which data/information can be exchanged between devices/apparatus/network.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable storage medium", "computer program product", "tangibly embodied computer program" etc., or a "processor" or "processing circuitry" etc. should be understood to encompass not only computers having differing architectures such as single/ multi-processor architectures and sequencers/parallel architectures, but also specialised circuits such as field programmable gate arrays FPGA, application specify circuits ASIC, signal processing devices/ apparatus and other devices/ apparatus. References to computer program, instructions, code etc. should be understood to express software for a programmable processor firmware such as the programmable content of a hardware device/ apparatus as instructions for a processor or configured or configuration settings for a fixed function device/ apparatus, gate array, programmable logic device/ apparatus, etc.

As used in this application, the term "circuitry" refers to all of the following: (a) hardware-only circuit implementations (such as implementations in only analogue and/or digital circuitry) and (b) to combinations of circuits and software (and/or firmware), such as (as applicable): (i) to a combination of processor(s) or (ii) to portions of processor(s)/ software (including digital signal processor(s)), software, and memory(ies) that work together to cause an apparatus, such as a server, to perform various functions) and (c) to circuits, such as a microprocessor(s) or a portion of a microprocessor(s), that require software or firmware for operation, even if the software or firmware is not physically present.

If desired, the different functions discussed herein may be performed in a different order and/or concurrently with each other. Furthermore, if desired, one or more of the above-described functions may be optional or may be combined. Similarly, it will also be appreciated that the flow diagram of FIG. 7 are examples only and that various operations depicted therein maybe omitted, reordered and/or combined.

It will be appreciated that the above described example embodiments are purely illustrative and are not limiting on the scope of the invention. Other variations and modifications will be apparent to persons skilled in the art upon reading the present specification.

Moreover, the disclosure of the present application should be understood to include any novel features or any novel combination of features either explicitly or implicitly disclosed herein or any generalization thereof and during the prosecution of the present application or of any application derived therefrom, new claims may be formulated to cover any such features and/or combination of such features.

## Claims

1. An apparatus comprising:
means for receiving one or more first signals indicative of an impact between a striking means and a tendon of a subject, caused by a strike by the striking means on the subject;
means for receiving one or more second signals indicative of a reflex response to said impact; and
means for generating a dataset based on one or more data elements obtained from the one or more first signals and the one or more second signals.

2. An apparatus as claimed in claim 1, wherein the one or more first signals are received from one or more first sensors and the one or more second signals are received from one or more second sensors.

3. An apparatus as claimed in claim 2, wherein the one or more first sensors comprise: an imaging device; one or more sensors attached to the subject; and/or form part of said striking means.

4. An apparatus as claimed in claim 2 or claim 3, wherein the one or more second sensors comprise one or more of: an imaging device; and one or more sensors attached to the subject.

5. An apparatus as claimed in any one of claims 2 to 4, wherein at least some of said sensors provide one or more of a timer data and a clock signal for recording time data associated with said signals.

6. An apparatus as claimed in any one of the preceding claims, wherein the one or more data elements comprise a first information of an impact location between the striking means and the tendon.

7. An apparatus as claimed in any one of the preceding claims, wherein the one or more data elements comprises a second information of impact force, number of strikes, time of one or more strikes and/or duration between respective strikes.

8. An apparatus as claimed in any one of the preceding claims, wherein the one or more data elements comprise a third information of speed of the striking means and/or direction of movement of striking means.

9. An apparatus as claimed in any one of the preceding claims, wherein the one or more data elements comprise a fourth information of a magnitude of the reflex response, a reflex location of the reflex response and/or a time of the reflex response.

10. An apparatus as claimed in any one of the preceding claims, wherein the one or more data elements comprise a fifth information of a duration between the impact and the reflex response.

11. An apparatus as claimed in any one of the preceding claims, further comprising means for transmitting said dataset.

12. An apparatus as claimed in any one of the preceding claims, further comprising means for storing said dataset.

13. An apparatus as claimed in any one of the preceding claims, wherein the means comprise:
at least one processor; and
at least one memory including computer program code, the at least one memory and the computer program configured, with the at least one processor, to cause the performance of the apparatus.

14. A method comprising:
receiving one or more first signals indicative of an impact between a striking means and a tendon of a subject, caused by a strike by the striking means on the subject;
receiving one or more second signals indicative of a reflex response to said impact; and
generating a dataset based on one or more data elements obtained from the one or more first signals and the one or more second signals.

15. A computer readable medium comprising program instructions stored thereon for performing at least the following:
receiving one or more first signals indicative of an impact between a striking means and a tendon of a subject, caused by a strike by the striking means on the subject;
receiving one or more second signals indicative of a reflex response to said impact; and
generating a dataset based on one or more data elements obtained from the one or more first signals and the one or more second signals.
